(19) **Europäisches Patentamt / European Patent Office / Office européen des brevets**

(11) **EP 4 778 648 A1**

(12) **DEMANDE DE BREVET EUROPEEN**

(43) Date de publication:
**22.07.2026 Bulletin 2026/30**

(21) Numéro de dépôt: **26152296.5**

(22) Date de dépôt: **16.01.2026**

(51) Classification Internationale des Brevets (IPC):
**B22D 17/00** (2006.01)    **B22D 17/22** (2006.01)
**A61B 17/00** (2006.01)

(52) Classification Coopérative des Brevets (CPC):
**B22D 17/007; B22D 17/2245;** A61B 34/30;
A61B 2017/00477; A61B 2017/00526;
A61B 2017/00831

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **17.01.2025 FR 2500506**

(71) Demandeur: **Vulkam**
**38610 Gières (FR)**

(72) Inventeurs:
• **GRAVIER, Sébastien**
**38420 MURIANETTE (FR)**
• **LENAIN, Alexis**
**38190 BERNIN (FR)**

(74) Mandataire: **Germain Maureau**
**12, rue Boileau**
**69006 Lyon (FR)**

(54) **PROCÉDÉ DE FABRICATION D'AU MOINS UNE PIÈCE EN UN VERRE MÉTALLIQUE**

(57) L'invention concerne un procédé de fabrication d'au moins une pièce moulée (2) en un verre métallique, comprenant une étape de mise à disposition (E1) d'un moule d'injection (1) délimitant une cavité (10) qui présente une surface texturée (s10), ladite surface texturée (s10) comprenant au moins un motif de structures miniatures (11), où chaque structure miniature (11) présente une portion sommitale (13) ; une étape d'injection (E3), dans laquelle un alliage métallique (3) à l'état liquide est injecté à l'intérieur de la cavité (10) ; et une étape de solidification (E4), dans laquelle l'alliage métallique (3) est moulé pour obtenir une pièce moulée (2) conformée selon la surface texturée (s10), ladite pièce moulée (2) présentant au moins partiellement une structure amorphe, et présentant à l'état solide, une limite élastique strictement supérieure à 2200 MPa.

L'invention concerne également une pièce moulée (2) et une plaquette de maintien (2a, 2b, 2c) comprenant une telle pièce moulée (2).

[Fig. 1]

## Description

### Domaine technique de l'invention

La présente invention concerne le domaine de la production par injection de pièces en verres métalliques, appelés aussi métaux amorphes ou alliages métalliques amorphes.

Plus particulièrement, l'invention concerne un procédé de fabrication d'au moins une pièce en un verre métallique, notamment pour la production de pièce pour outillage chirurgical robotisé pour la préhension d'outil.

### Etat de la technique

Dans le domaine de la chirurgie, les praticiens peuvent être amenés à utiliser de l'outillage robotisé ou des systèmes mécanisés pour réaliser les opérations. De tels robots utilisent des outils de très petite taille maintenus au niveau d'embouts du robot. Ces embouts sont généralement munis de plaquettes texturées permettant de faciliter la préhension des outils de petite taille.

Les plaquettes en question contiennent généralement une matrice de pointes pyramidales permettant de maintenir un micro-outil entre deux plaquettes en vis-à-vis. Pour cela, la géométrie de cette pointe pyramidale doit être extrêmement précise afin de se positionner parfaitement en vis-à-vis.

Afin de réaliser de telles plaquettes, il est connu de l'état de la technique d'utiliser un matériau très dur comme le carbure de tungstène ou l'acier trempé.

Pour assurer un bon maintien du micro-outil, il faut donc maximiser la friction avec la plaquette et donc l'effort normal appliqué (via le coefficient de frottement), ce qui génère une pression de Hertz qui déforme les pointes pyramidales, sans endommager la structure matricielle. L'utilisation de matériaux très durs (donc généralement de faible ténacité) couplé à la géométrie précise et fine engendrent des problématiques de pression de Hertz très importantes et localisées, entrainant des risques de rupture ou d'endommagement des pointes.

De plus, les procédés de fabrication conventionnels pour obtenir ce type de géométrie (usinage) sont complexes, et limitent les possibilités géométriques, notamment vis-à-vis des rayons de congé des creux et des pointes de la pyramide.

Alternativement, il est connu de l'état de la technique, d'utiliser des procédés de frittage de poudre. Bien que ces procédés donnent satisfaction en ce qu'ils permettent d'obtenir les plaquettes, ils génèrent des géométries moins précises.

Par ailleurs, les risques de rupture de parties d'outils font peser un risque pour le patient car les morceaux détachés peuvent rester dans le corps du patient. Dans le cas de la métallurgie des poudres et du frittage, il est possible que de la poudre se détache, notamment aux alentours des pointes où les poudres sont moins bien frittées. Dans le cas des matériaux en carbure de tungstène, des éclats peuvent contenir des composants néfastes pour la santé tel que du Cobalt.

Il existe donc un besoin de trouver un procédé de fabrication d'une pièce métallique adapté pour la fabrication de plaquette texturée pour outillage chirurgical, qui permette d'obtenir des pièces extrêmement dures, ayant une bonne ténacité, et présentant des propriétés mécaniques les rendant très résistantes aux pressions de Hertz.

### Objet de l'invention

La présente invention a pour but de proposer une solution qui réponde à tout ou partie des problèmes précités.

Ce but peut être atteint grâce à la mise en oeuvre d'un procédé de fabrication d'au moins une pièce moulée en un verre métallique, comprenant les étapes suivantes :

- une étape de mise à disposition d'un moule d'injection délimitant une cavité qui présente une surface texturée, ladite surface texturée comprenant au moins un motif de structures miniatures, où chaque structure miniature présente une portion sommitale ;
- une étape d'injection, dans laquelle un alliage métallique à l'état liquide est injecté à l'intérieur de la cavité ;
- une étape de solidification, dans laquelle l'alliage métallique est moulé pour obtenir une pièce moulée conformée selon la surface texturée, ladite pièce moulée présentant au moins partiellement une structure amorphe, ladite pièce moulée présentant une limite élastique strictement supérieure à 2200 MPa.

Les dispositions précédemment décrites permettent de proposer un procédé de fabrication d'une pièce moulée présentant une surface ayant un motif régulier de structures de préhension miniatures, ladite pièce moulée ayant en outre une structure au moins partiellement amorphe. De cette manière, il est possible de produire une pièce qui possède directement les caractéristiques géométriques et fonctionnelles sans étapes supplémentaires tels que des traitements thermiques ou de surface.

De manière générale, la limite élastique de la pièce moulée est mesurée en flexion.

Par « structure miniature », on entend que chaque structure est de taille micrométrique, c'est-à-dire qu'elle présente une plus grande dimension inférieure à 2 mm.

Le procédé de fabrication peut en outre présenter une ou plusieurs des caractéristiques suivantes, prises seules ou en combinaison.

Selon un mode de réalisation, la limite d'élasticité de la pièce moulée est strictement supérieure à 2400 MPa, ou strictement supérieure à 2800 MPa.

Ainsi, la pièce moulée sera apte à résister à de fortes pressions de Hertz, ce qui est particulièrement avantageux pour des pièces de petites tailles, notamment celles utilisées dans le domaine de l'outillage robotisé.

Selon un mode de réalisation, lors de l'étape d'injection, l'alliage métallique est un alliage comprenant une base métallique en Nickel, en Fer, en Cobalt, en Hafnium ou en Titane.

De manière avantageuse, l'utilisation de ces types d'alliages métalliques permet d'obtenir des matériaux au moins partiellement amorphes présentant une limite d'élasticité haute. En particulier, les alliages métalliques à base de fer et cobalt présentent une haute limite élastique, ce qui leur permet de résister à de fortes pressions de Hertz. Ils présentent par ailleurs une rigidité plus faible que le carbure de tungstène généralement utilisé.

Les alliages à base de nickel, hafnium et titane présentent une limite élastique plus basse, mais sont moins fragiles et se déforment si la pression de Hertz appliquée est supérieure à leur limite élastique.

Selon un mode de réalisation, l'alliage métallique comprend un alliage Nickel/Niobium.

De manière avantageuse, l'alliage Nickel/Niobium permet d'obtenir une pièce moulée ayant de très bonnes caractéristiques de résistance, de dureté et de ténacité tout en ayant une rigidité plus faible que les matériaux cristallins utilisés actuellement, limitant ainsi les pressions de Hertz sur les structures miniatures. Les alliages à base de nickel présentent donc un excellent compromis en termes de limite élastique et de fragilité (haute limite élastique et moins grande fragilité).

Selon un mode de réalisation, la pièce moulée présente un module d'Young inférieur à 400 GPa, préférentiellement inférieure à 200 GPa, et préférentiellement inférieur à 150 GPa.

Selon un mode de réalisation, lors de l'étape de mise à disposition, la portion sommitale de chaque structure miniature présente un rayon de courbure inférieur à 300 $\mu$m.

Le procédé est ainsi adapté pour former une pièce moulée présentant une surface ayant une pluralité de structures miniatures adaptées pour la manipulation de précision. Par ailleurs, l'utilisation d'un moule d'injection pour former la pièce permet de définir plus précisément la géométrie des creux de la pièce moulée (correspondant aux parties sommitales de la surface texturée du moule d'injection). L'usinage des pointes de la surface texturée du moule d'injection étant plus simple à mettre en œuvre que l'usinage des creux d'une pièce métallique ayant ces **dimensions.** Ainsi, le procédé de fabrication est particulièrement adapté pour des pièces entrant en contact avec des outils de précision, les creux formés dans le motif de structures miniatures de la pièce moulée étant déterminants pour fiabiliser la préhension des outils de précision.

Généralement, la pointe des structures miniatures sur les outils de précisions est la partie la plus fragile et la plus susceptible de casser. L'utilisation d'un verre métallique pour former les portions sommitales est donc particulièrement adapté pour limiter les risques de casse.

Selon un mode de réalisation, le procédé de fabrication comprend en outre une étape de découpe, dans laquelle la pièce moulée solidifiée issue de l'étape de solidification est découpée.

Ainsi, il est possible de réalisation une préforme de grande dimension, présentant une surface micro texturée, et de découper ensuite cette préforme aux dimensions adaptées à l'application pour obtenir la pièce finale.

Par exemple, l'étape de découpe est mise en œuvre par découpe jet d'eau, par découpe laser femto seconde, ou par électroérosion (ou EDM de l'anglais « electrodischarge machining »).

La mise en œuvre d'une étape de découpe est particulièrement intéressante pour s'adapter à la forme des outils à réaliser. En effet, le procédé de fabrication est adapté pour former une préforme en un verre métallique, et l'étape de découpe permet de découper cette préforme pour réaliser une plaquette d'un outil particulier. Cela permet d'utiliser un seul procédé pour réaliser une multitude de pièces ayant des géométries différentes. Le procédé est ainsi simplifié et présente un cout plus faible.

Selon un mode de réalisation, le procédé de fabrication comprend en outre une étape de fusion, mise en œuvre avant l'étape d'injection, dans laquelle l'alliage métallique est fondu par induction pour obtenir ledit alliage métallique à l'état liquide.

L'utilisation d'un chauffage par induction est particulièrement adaptée pour la réalisation de pièce métallique au moins partiellement amorphe. En effet, ce type de chauffage permet d'obtenir une bonne homogénéité lors de la fusion, cela permet d'obtenir la qualité de métal fondu suffisante pour la réalisation d'une pièce métallique au moins partiellement amorphe. Il est toutefois possible d'utiliser d'autres modes de fusion comme un arc électrique, un laser ou un faisceau d'électron.

Selon un mode de réalisation, lors de l'étape d'injection, la cavité du moule d'injection est maintenue sous vide, ou en présence d'un gaz inerte comme l'argon. Par exemple, la pression interne régnant à l'intérieur de la cavité du moule d'injection est maintenue inférieure à 1 mbar, préférentiellement à 0,01 mbar, préférentiellement à 0.0001 mbar, avec 1 bar égal à $10^5$ Pa.

Cela permet d'obtenir une qualité métallurgique de l'alliage métallique suffisante pour obtenir une structure au moins partiellement amorphe.

Selon un mode de réalisation, lors de l'étape d'injection, un temps de remplissage de la cavité du moule d'injection par l'alliage métallique à l'état liquide est inférieur à 1 s, préférentiellement inférieur à 100 ms, et préférentiellement inférieur à 50 ms. Il est ainsi possible de maîtriser la vitesse d'écoulement de l'alliage métallique dans la cavité du moule d'injection.

La maitrise de la vitesse d'écoulement lors de l'étape d'injection permet de bien remplir la cavité de manière répétable notamment au niveau de la surface texturée, ce qui permet d'obtenir de meilleures caractéristiques géométriques. La pièce moulée ainsi obtenue présente une surface dont les structures miniatures sont définies plus précisément.

En effet, une vitesse d'écoulement trop faible ne permettrait pas un bon remplissage de la cavité du moule d'injection.

Selon un mode de réalisation, lors de l'étape d'injection, l'alliage métallique à l'état liquide est injecté à une pression supérieure à 10 MPa et notamment supérieure à 50 MPa.

La maitrise de la pression d'injection lors de l'étape d'injection, et notamment sur la fin de l'injection, permet de bien remplir la cavité notamment au niveau de la surface texturée, ce qui permet d'obtenir de meilleures caractéristiques géométriques. La pièce moulée ainsi obtenue présente une surface dont les structures miniatures sont définies plus précisément.

Selon un mode de réalisation, l'au moins un motif de structures miniatures est un motif régulier de structures miniatures.

Selon un mode de réalisation, lors de l'étape de mise à disposition, le motif régulier de la surface texturée comprend l'alternance de creux et de pointes, lesdites pointes formant les portions sommitales.

Ainsi, il est possible de former, par complémentarité, une pièce moulée présentant une surface de préhension texturée formée de creux (correspondant aux pointes du moule d'injection) et de pointes (correspondant aux creux du moule d'injection). Dans ce cas, l'utilisation d'une pièce moulée ayant une structure au moins partiellement amorphe permet de limiter les pressions de Hertz sur les pointes.

Selon un mode de réalisation, le motif régulier comprend une matrice de pyramides.

Ainsi, la formation de la surface texturée est facilitée.

Selon un mode de réalisation, lors de l'étape de mise à disposition, le motif de structures miniatures comprend une face plane au niveau de laquelle la pluralité de structures miniatures fait saillie régulièrement de ladite face plane.

Ainsi, il est possible d'obtenir une pièce moulée de forme généralement plane, plus simple à découper lors d'une post-opération après le moulage.

Le but de l'invention peut également être atteint grâce à la mise en oeuvre d'une pièce moulée en un verre métallique présentant au moins partiellement une structure amorphe, ladite pièce moulée présentant à l'état solide, une limite élastique strictement supérieure à 2200 MPa, ladite pièce moulée présentant en outre une surface de préhension texturée comprenant au moins un motif de structures de préhension miniatures, où chaque structure de préhension miniature présente une portion creuse.

Selon un mode de réalisation, la pièce moulée présente une capacité de déformation élastique d'au moins 1,2%, et préférablement d'au moins 1,5%.

Selon un mode de réalisation, la pièce moulée présente une contribution plastique à la flèche fp, supérieure à 0 mm, préférentiellement supérieure à 0,25 mm, plus préférentiellement supérieure à 0,60 mm, encore plus préférentiellement supérieure à 1 mm, ladite contribution plastique à la flèche fp étant déterminée lors d'un essai de flexion 3 points dans la direction de l'épaisseur pour un échantillon d'épaisseur 0,5 mm, de largeur 10 mm et de longueur 15 mm, une longueur entre appuis de 10 mm et une vitesse de traverse de 0,005 mm/s.

L'invention concerne également une plaquette de maintien pour outillage robotisé comprenant une pièce moulée telle que décrite précédemment.

**Description sommaire des dessins**

D'autres aspects, buts, avantages et caractéristiques de l'invention apparaîtront mieux à la lecture de la description détaillée suivante de modes de réalisation préférés de celle-ci, donnée à titre d'exemple non limitatif, et faite en référence aux dessins annexés sur lesquels :

[Fig. 1] La figure 1 est une vue schématique des premières étapes du procédé de fabrication selon un mode de réalisation particulier de l'invention.
[Fig. 2] La figure 2 est une vue schématique de l'étape d'extraction selon un mode de réalisation particulier de l'invention.
[Fig. 3] La figure 3 est une vue schématique de l'étape de découpe selon un mode de réalisation particulier de l'invention.
[Fig. 4] La figure 4 est un graphe présentant une comparaison entre des pièces de l'état de la technique et une pièce moulée selon l'invention.
[Fig. 5] La figure 5 représente une analyse par diffraction X d'un alliage métallique amorphe.
[Fig. 6] La figure 6 représente une analyse par diffraction X d'un alliage métallique partiellement amorphe.
[Fig. 7] La figure 7 représente une analyse par diffraction X d'un alliage métallique cristallin.

**Description détaillée**

Sur les figures et dans la suite de la description, les mêmes références représentent les éléments identiques ou similaires. De plus, les différents éléments ne sont pas représentés à l'échelle de manière à privilégier la clarté des figures. Par ailleurs, les différents modes de réalisation et variantes ne sont pas exclusifs les uns des autres et peuvent être combinés entre eux.

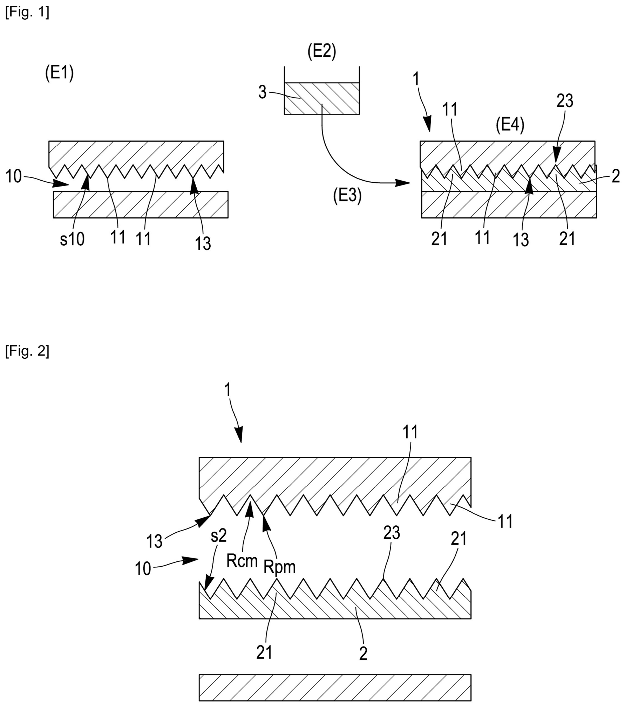

Comme on peut le voir sur les figures 1 à 3, l'invention concerne un procédé de fabrication d'au moins une pièce moulée 2 en un verre métallique. L'invention concerne également une telle pièce moulée 2, ainsi qu'une plaquette de maintien 2a, 2b, 2c pour outillage robotisé comprenant une telle pièce moulée 2. Bien que cela ne soit pas limitatif, de telles plaquettes de maintien 2a, 2b, 2c sont particulièrement adaptées pour l'outillage robotisé dans le domaine de la chirurgie, notamment pour la préhension d'outils de chirurgie.

Le procédé de fabrication comprend tout d'abord une étape de mise à disposition E1 d'un moule d'injection 1.

Le moule d'injection 1 délimite intérieurement une cavité 10 qui présente une surface texturée s10. Pour simplifier les figures, seulement une partie du moule d'injection 1 a été représentée pour illustrer les éléments nécessaires à la compréhension de l'invention. La surface texturée s10 de la cavité 10 comprend au moins un motif de structures miniatures 11. Par « structure miniature 11 », on entend que chaque structure 11 est de taille micrométrique, c'est-à-dire qu'elle présente une plus grande dimension inférieure à 2 mm. Sur le mode de réalisation représenté, la surface texturée ne comprend qu'un seul motif comprenant dix structures miniatures 11. Il est cependant bien entendu que suivant l'application visée, plusieurs motifs de structures miniatures 11 peuvent être inclus sur la surface texturée s10 de la cavité, et que ces motifs peuvent comprendre n'importe quel nombre de structures miniatures 11.

Par ailleurs, il est possible que l'au moins un motif de structures miniatures 11 soit un motif régulier de structures miniatures 11. Par exemple, le motif régulier de structures miniatures 11 peut présenter une répartition matricielle de structures miniatures 11. La disposition sur la surface texturée s10 d'une pluralité de structures miniatures 11 qui se répètent est particulièrement difficile à produire par des procédés d'usinage classiques, notamment à des dimensions inférieures au millimètre.

Comme on peut le voir sur les figures, chaque structure miniature 11 présente une portion sommitale 13. Par exemple, dans le cas d'un motif régulier, il est possible de prévoir que ce motif comprenne une alternance de creux et de pointes, lesdites pointes formant les portions sommitales 13. Ainsi, il est possible de former, par complémentarité, une pièce moulée 2 présentant une surface de préhension texturée s2 formée de creux (correspondant aux pointes du moule d'injection 1) et de pointes (correspondant aux creux du moule d'injection 1).

Selon un mode de réalisation, la portion sommitale 13 de chaque structure miniature 11 présente un rayon de courbure Rpm inférieur à 300 $\mu$m, préférentiellement inférieur à 150 $\mu$m, 100 $\mu$m, 80 $\mu$m, 50 $\mu$m ou 40 $\mu$m. Le procédé est ainsi adapté pour former une pièce moulée 2 présentant une surface ayant une pluralité de structures miniatures 11 adaptées pour la manipulation de précision.

Dans le cas où le motif régulier de la surface texturée s10 comprend une alternance de creux et de pointes, il est également possible de prévoir que les creux aient un rayon de courbure Rcm inférieur à 300 $\mu$m, préférentiellement inférieur à 150 $\mu$m, 100 $\mu$m, 80 $\mu$m, 50 $\mu$m ou 40 $\mu$m. Ainsi, les rayons de courbure Rcm dans les creux peuvent être du même ordre de grandeur (identiques ou différents) que les rayons de courbure Rpm au niveau des pointes.

De manière avantageuse, l'utilisation d'un moule d'injection 1 pour former la pièce moulée 2 permet de définir plus précisément la géométrie des creux Rcm de la pièce moulée 2 (correspondant aux parties sommitales 13 de la surface texturée s10 du moule d'injection 1). L'usinage des pointes de la surface texturée s10 du moule d'injection 1 étant plus simple à mettre en œuvre que l'usinage des creux d'une pièce métallique ayant ces dimensions. Ainsi, le procédé de fabrication est particulièrement adapté pour des pièces entrant en contact avec des outils de précision, les creux formés dans le motif de structures miniatures 11 de la pièce moulée 2 étant déterminants pour fiabiliser la préhension des outils de précision.

Selon un mode de réalisation, le motif régulier peut comprendre une matrice de pyramides. Ainsi, la formation de la surface texturée s10 est facilitée. Une telle variante n'est cependant pas limitative et il est tout à fait possible que les structures miniatures 11 comprennent des bosses ou des créneaux, ou tout autre élément faisant saillie de la surface texturée s10. Il est donc bien compris qu'une portion sommitale 13 n'est pas réduite à une portion formant un angle aigu, et que les structures miniatures 11 sont des aspérités de surface, identiques ou différentes, et faisant saillie de la surface texturée s10.

Le procédé de fabrication comprend généralement une étape de fusion E2, dans laquelle un alliage métallique 3 est fondu par induction pour obtenir cet alliage métallique 3 à l'état liquide. L'utilisation d'un chauffage par induction est particulièrement adaptée pour la réalisation de pièce métallique au moins partiellement amorphe. Il est toutefois possible d'utiliser d'autres modes de fusion comme un arc électrique, un laser ou un faisceau d'électron.

Par exemple l'alliage métallique 3 peut comprendre une base métallique en Nickel, en Fer, en Cobalt, ou en Hafnium. De manière avantageuse, l'utilisation de ces types d'alliages métalliques permet d'obtenir des matériaux au moins partiellement amorphes présentant une limite d'élasticité haute. En particulier, les alliages métalliques à base de fer et cobalt présentent une haute limite élastique, ce qui leur permet de résister à de fortes pressions de Hertz. Les alliages à base de nickel et hafnium présentent une limite élastique plus basse, mais sont moins fragiles. Ils peuvent ainsi se déformer si la pression de Hertz appliquée est supérieure à leur limite élastique.

La pression de Hertz maximum initiale $P_0$ lors d'un contact entre deux objets formés par le même matériau est définie par la formule F1 suivante :

$$P_O = \frac{1}{\pi}\left(\frac{3}{2}\right)^{1/3} \times F_N^{1/3} \times \left(\frac{E}{1-\nu^2}\right)^{2/3} \times R_{equiv}^{-2/3} \quad , \text{ où}$$

$F_N$ est la force normale appliquée entre les deux objets,

$R_{equiv}$ est défini par la formule F2 suivante :

$$R_{\text{équiv}} = \frac{R_{pm} \cdot R_{cm}}{R_{pm} + R_{cm}}$$ , E est le module d'Young, et v est le coefficient de Poisson.

Plus précisément, il est possible d'utiliser un alliage Nickel/ Niobium. Un tel alliage Nickel/Niobium permet d'obtenir une pièce moulée 2 ayant de très bonnes caractéristiques de résistance, de dureté et de ténacité tout en ayant une rigidité plus faible que les matériaux cristallins, limitant ainsi les pressions de Hertz sur les structures miniatures 11 (voir formule F1). Les alliages à base de nickel présentent donc un excellent compromis en termes de limite élastique et de fragilité (haute limite élastique et moins grande fragilité).

Suite à cette étape de fusion E2, le procédé de fabrication comprend une étape d'injection E3, dans laquelle l'alliage métallique 3 à l'état liquide est injecté à l'intérieur de la cavité 10.

De manière avantageuse, lors de l'étape d'injection E3, la cavité 10 du moule d'injection 1 peut être maintenue sous vide, ou en présence d'un gaz inerte comme l'argon. Par exemple, la pression interne régnant à l'intérieur de la cavité 10 du moule d'injection 1 est maintenue inférieure à 1 mbar, préférentiellement à 0,01 mbar, préférentiellement à 0.0001 mbar, avec 1 bar égal à $10^5$ Pa. Cela permet d'obtenir une qualité métallurgique de l'alliage métallique 3 suffisante pour obtenir une structure au moins partiellement amorphe.

Par ailleurs, il est possible de contrôler les paramètres d'introduction de l'alliage métallique 3 à l'intérieur de la cavité pour adapter les propriétés de la pièce moulée 2 obtenue à la fin du procédé. Pour cela, il est par exemple possible de prévoir qu'un temps de remplissage de la cavité 10 du moule d'injection 1 par l'alliage métallique 3 à l'état liquide est inférieur à 1 s, préférentiellement inférieur à 100 ms, et préférentiellement inférieur à 50 ms. Il est ainsi possible de maîtriser la vitesse d'écoulement de l'alliage métallique 3 dans la cavité 10 du moule d'injection 1.

. La maitrise de la vitesse d'écoulement de l'alliage métallique 3 lors de l'étape d'injection E3 permet de remplir la cavité 10 de manière répétable notamment au niveau de la surface texturée s10, ce qui permet d'obtenir de meilleures caractéristiques géométriques. La pièce moulée 2 ainsi obtenue présente une surface dont les structures miniatures 11 sont définies plus précisément. En effet, une vitesse d'écoulement trop faible ne permettrait pas un bon remplissage de la cavité 10 du moule d'injection 1.

Un autre paramètre peut être utilisé pour contrôler la mise en œuvre de l'étape d'injection : la pression d'injection. Par exemple, lors de l'étape d'injection E3, l'alliage métallique 3 à l'état liquide est injecté à une pression supérieure à 10 MPa et notamment supérieure à 50 MPa. La maitrise de la pression d'injection lors de l'étape d'injection E3, et notamment sur la fin de l'injection permet de bien remplir la cavité 10 notamment au niveau de la surface texturée s10, ce qui permet d'obtenir de meilleures caractéristiques géométriques. La pièce moulée 2 ainsi obtenue présente une surface dont les structures miniatures 11 sont définies plus précisément. L'étape d'injection E3 peut par exemple être mise en œuvre par un piston permettant l'application d'un différentiel de pression et faciliter le remplissage de la cavité 10 du moule d'injection 11. Cela permet d'assurer un contact intime entre l'alliage métallique 3 et les parois de la cavité 10 du moule d'injection 11 afin d'assurer un refroidissement rapide recherché de la pièce moulée 2. La pression d'injection permet d'injecter l'alliage métallique 3 à l'état liquide dans la cavité 10.

Le procédé comprend ensuite une étape de solidification E4, dans laquelle l'alliage métallique 3 est moulé pour obtenir une pièce moulée 2 conformée selon la surface texturée s10. En effet, la température du moule d'injection1 induit un refroidissement rapide et une solidification de l'alliage métallique 3 injecté dans la cavité 10. La pièce moulée 2 ainsi obtenue présente au moins partiellement une structure amorphe et présente une limite élastique strictement supérieure à 2200 MPa. De manière générale, la limite élastique de la pièce moulée 2 est mesurée en flexion. Selon un mode de réalisation, la limite d'élasticité de la pièce moulée 2 est strictement supérieure à 2400 MPa, ou strictement supérieure à 2800 MPa. Ainsi, la pièce moulée 2 sera apte à résister à de fortes pressions de Hertz, ce qui est particulièrement avantageux pour des pièces de petites tailles, notamment celles utilisées dans le domaine de l'outillage robotisé. Par ailleurs, la pièce moulée 2 présente généralement un module d'Young inférieur à 400 GPa, préférentiellement inférieure à 200 GPa, et préférentiellement inférieur à 150 GPa.

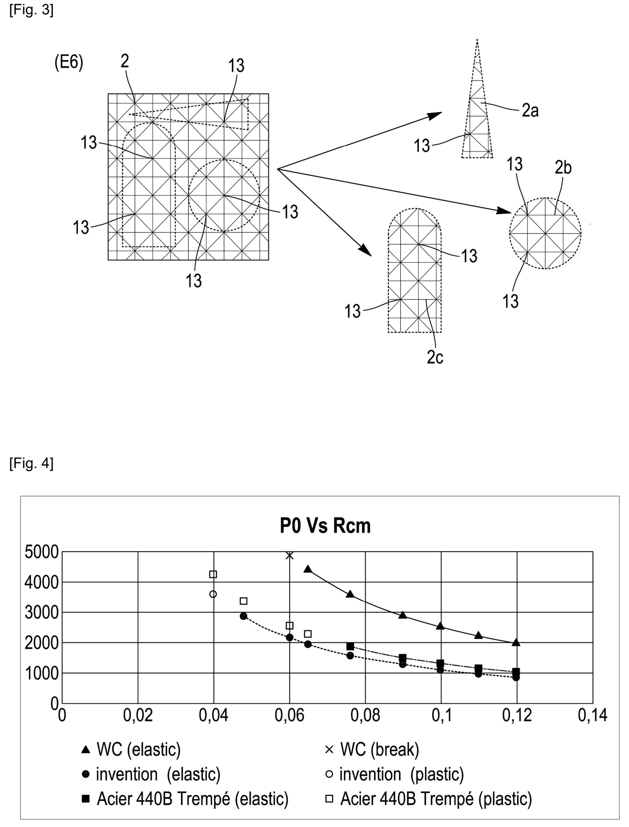

La formation d'une pièce moulée 2 ayant une structure au moins partiellement amorphe permet de limiter les pressions de Hertz sur les pointes. Généralement, la pointe des structures miniatures 11 sur les outils de précisions est la partie la plus fragile et la plus susceptible de casser. L'utilisation d'un verre métallique pour former les portions sommitales 13 est donc particulièrement adapté pour limiter les risques de casse. La figure 4 illustre les performances améliorées d'une pièce moulée 2 selon l'invention, par rapport à des pièces de l'état de la technique en acier trempé, ou en carbure de tungstène.

Une pièce présentant « au moins partiellement une structure amorphe » ou « une structure au moins partiellement amorphe » peut être définie ou comprise selon les définitions ci-dessous. L'homme du métier peut également se référer à l'ouvrage de référence « Suryanarayana, C., & Inoue, A. (2017). Bulk Metallic Glasses (2nd ed.). CRC Press. » concernant les matériaux au moins partiellement amorphes.

On entend par « sphères géométriques inscrites » les sphères géométriques dont les diamètres maximums sont tels qu'elles sont coincées ou immobilisées entre des points des parois de la cavité 10.

On entend par « diamètre critique » Dc d'un alliage métallique spécifique l'épaisseur limite maximum en deçà de laquelle l'alliage métallique présente une structure métallurgique entièrement amorphe ou au-delà de laquelle **il** n'est plus possible d'obtenir une structure métallurgique entièrement amorphe, lorsque l'alliage métallique est moulé depuis un état liquide et est soumis à un refroidissement rapide tel que le transfert de la chaleur à l'intérieur de l'alliage métallique soit optimal.

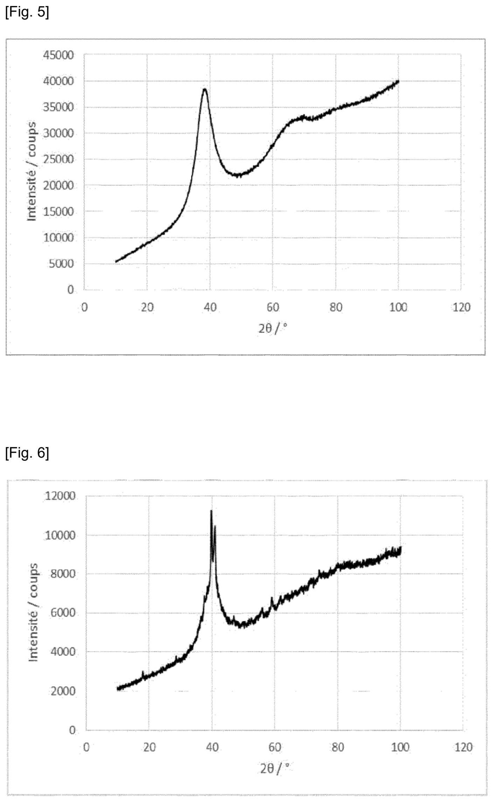

On précise ici qu'une structure métallurgique est dite « totalement amorphe » au sens de la présente invention lorsqu'une analyse par diffraction des rayons X telle que décrite ci-dessous ne met pas en évidence de pic de cristallisation comme ceci est illustré à la figure 5. Une structure métallurgique est dite « au moins partiellement amorphe » ou « partiellement amorphe » au sens de la présente invention lorsqu'une analyse par diffraction des rayons X telle que décrite ci-dessous met en évidence quelques pics de cristallisation comme ceci est illustré à la figure 6. Le terme « amorphe » est utilisé tant pour les alliages dits « totalement amorphes » que pour les alliages dits « partiellement amorphes » au sens de l'invention.

Une telle évaluation du caractère amorphe d'un alliage métallique est détaillée dans l'article Cheung et al., 2007 (Cheung et al. (2007) «Thermal and mechanical properties of Cu-Zr-Al bulk metallic glasses) » doi:10.10161/jallcom.2006.08.109). Elle permet de faire une analyse moyenne sur une surface et de s'affranchir des quelques défauts métallurgiques inévitables, tout en analysant uniquement les cristaux de taille significative, c'est-à-dire supérieure à quelques nanomètres et/ou en quantité significative. Les figures 5 à 7 représentent une analyse DRX telle que décrite précédemment. Ces figures montrent l'intensité du faisceau diffracté en fonction de l'angle entre le faisceau incident et le faisceau diffracté.

La figure 5 est une analyse DRX d'un alliage métallique à l'état « totalement amorphe », la fraction amorphe étant très largement majoritaire par rapport à la fraction cristalline.

La figure 6 est une analyse similaire réalisée sur un alliage à l'état « partiellement amorphe », la fraction amorphe étant majoritaire par rapport à la fraction cristalline. Sur cette figure, on retrouve la bosse caractéristique des structures amorphes, mais avec la présence également de pics.

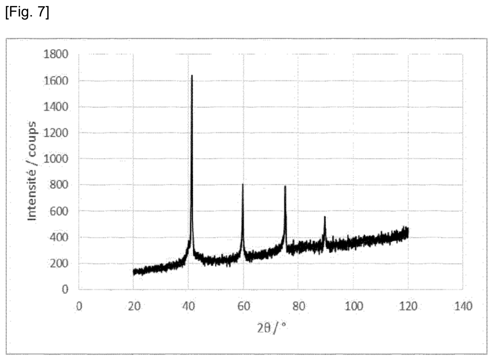

La figure 7 est une analyse similaire réalisée sur un alliage cristallin, la fraction cristalline étant majoritaire par rapport à la fraction amorphe. Sur la figure 7, les pics de cristallinité sont bien visibles.

Une méthode de détermination du diamètre critique Dc d'un alliage métallique consiste à fabriquer, par exemple successivement, des barreaux cylindriques de diamètres différents dans des empreintes correspondantes d'un moule sous les conditions suivantes.

Le moule est en une matière présentant des propriétés thermiques spécifiques telles qu'une conductivité thermique, une effusivité et une diffusivité élevées par exemple en cuivre et est refroidi à une température maximum d'environ vingt degrés Celsius 20°C.

L'alliage métallique est fondu avec une surfusion d'au moins environ deux cents degrés 200°C puis moulé sous l'effet d'un système. Par exemple un piston permettant l'application d'un différentiel de pression pour faciliter le remplissage de l'empreinte du moule et assurer un contact intime entre l'alliage métallique et les parois de l'empreinte du moule afin d'assurer le refroidissement rapide recherché du barreau moulé.

Avantageusement, l'alliage métallique est élaboré et moulé sous atmosphère inerte et de haute pureté.

Après quoi, les barreaux sont coupés transversalement, par tronçonnage ou sciage, en limitant les échauffements, afin d'obtenir des tranches. Celles-ci sont alors préparées polissage. Puis, Il est procédé à des analyses métallurgiques, par exemple sous microscope optique, microscope électronique à balayage et diffraction de Rayons X, de sorte à déterminer, pour chaque tranche, la présence d'une structure métallurgique entièrement amorphe ou la présence d'une structure amorphe et partiellement cristalline.

Etant donné que, dans la pratique, il existe toujours des défauts dans les structures métallurgiques, un alliage cent pour cent amorphe est quasi impossible à obtenir. C'est pourquoi, le diamètre critique Dc de l'alliage métallique analysé peut être déterminé comme étant celui du barreau de plus grand diamètre, présentant éventuellement des défauts métallurgiques et tel que pour les barreaux de plus grands diamètres, une analyse par diffraction des rayons X met clairement en évidence des pics de cristallinité qui montrent la présence d'une quantité significative de cristaux généralement supérieures à quelques nanomètres.

Ensuite, et comme cela est illustré sur la figure 2, le procédé de fabrication peut comprendre une étape d'extraction de la pièce moulée 2 hors du moule d'injection 1. Pour cela, le moule d'injection peut être désassemblé, notamment si celui-ci comprend plusieurs parties de moule, et la pièce moulée 2 est extraite.

Selon un variante non limitative représentée sur la figure 3, le procédé de fabrication peut comprendre une étape de découpe E6, dans laquelle la pièce moulée 2 solidifiée issue de l'étape de solidification E4 est découpée. La pièce moulée peut alors être considérée comme une préforme de grande dimension, présentant une surface micro texturée, qui est ensuite découpée à des dimensions adaptées à l'application, pour obtenir la pièce finale 2a, 2b, 2c. Par exemple, l'étape de découpe E6 est mise en œuvre par découpe jet d'eau, par découpe laser femto seconde, par fraisage ou par électroérosion (ou EDM de l'anglais « electrodischarge machining »). La mise en œuvre d'une étape de découpe E6 est particulièrement intéressante pour s'adapter à la forme des outils à réaliser. En effet, le procédé de fabrication est adapté pour former une préforme en un verre métallique, et l'étape de découpe E6 permet de découper cette préforme pour réaliser une plaquette d'un outil particulier. Cela permet d'utiliser un seul procédé pour réaliser une multitude de pièces ayant des géométries différentes. Le procédé est ainsi simplifié et présente un cout plus faible.

Alternativement, il est possible que la cavité 10 du moule d'injection 1 soit conformée directement pour être une empreinte de la pièce finale 2a, 2b, 2c. Dans ce cas, il n'est pas nécessaire de mettre en œuvre l'étape de découpe E6, la pièce moulée 2 obtenue à l'issue de l'étape de solidification E4 peut correspondre à la pièce finale 2a, 2b, 2c.

Les dispositions précédemment décrites permettent de proposer un procédé de fabrication d'une pièce moulée 2 présentant une surface ayant un motif régulier de structures de préhension miniatures, ladite pièce moulée 2 ayant en outre une structure au moins partiellement amorphe. De cette manière, il est possible de produire une pièce qui possède directement les caractéristiques géométriques matrice de creux et fonctionnelles dureté, résistance, ténacité sans étapes supplémentaires tels que des traitements thermiques ou de surface.

Cette pièce moulée 2 en un verre métallique présente au moins partiellement une structure amorphe, et une surface de préhension texturée s2 comprenant au moins un motif de structures de préhension miniatures 21, où chaque structure de préhension miniature 21 présente une portion creuse 23. Par exemple, la pièce moulée 2 peut présenter une capacité de déformation élastique d'au moins 1,2%, et notamment d'au moins 1,5%.

Les pièces moulée 2 en un verre métallique amorphe selon l'invention présentent avantageusement une contribution plastique à la flèche, fp, évaluée lors d'un essai de flexion 3 points dans la direction de l'épaisseur pour un échantillon d'épaisseur 0,5 mm, de largeur 10 mm et de longueur 15 mm, une longueur entre appuis de 10 mm et une vitesse de traverse de 0,005 mm/s, supérieure à 0 mm, préférentiellement supérieure à 0,25 mm, plus préférentiellement supérieure à 0,60 mm, encore plus préférentiellement supérieure à 1 mm.

Selon la présente description, la limite élastique, $\sigma_{el}$, et la contribution plastique à la flèche, fp, sont évaluées comme suit.

Les essais mécaniques sont réalisés sur une machine d'essai mécanique DY34 (Adamel Lhomargy). Ce sont des essais de flexion 3 points dans la direction de l'épaisseur de l'échantillon.

Les paramètres de l'essai sont les suivants :

- Longueur entre appuis L=10 mm
- Largeur échantillon b=10 mm
- Epaisseur échantillon h=0,50 mm
- Longueur échantillon l=15 mm
- Vitesse de traverse v=0,005 mm/s

La courbe de flexion 3 points présente une première partie élastique linéaire, puis un plateau plastique.

La limite élastique, $\sigma_{el}$, est calculée selon la formule suivante :

$$\sigma_{el}=(3\times Fe\times L)/(2\times b\times h^2) ;$$

où Fe est calculé selon la formule suivante :

$$Fe=2Fmax/3 ;$$

où Fmax est la valeur de force maximale enregistrée au niveau du plateau de force.

La contribution plastique à la flèche, fp, est calculée selon la formule suivante :

$$fp=fr-fe ;$$

où fe est la flèche atteinte à un niveau de force correspondant à la limite élastique, soit 2Fmax/3 ; et où fr est la flèche à rupture.

## Revendications

1. Procédé de fabrication d'au moins une pièce moulée (2) en un verre métallique, comprenant les étapes suivantes :

   - une étape de mise à disposition (E1) d'un moule d'injection (1) délimitant une cavité (10) qui présente une surface texturée (s10), ladite surface texturée (s10) comprenant au moins un motif de structures miniatures (11), où chaque structure miniature (11) présente une portion sommitale (13) ;
   - une étape d'injection (E3), dans laquelle un alliage métallique (3) à l'état liquide est injecté à l'intérieur de la cavité (10) ;
   - une étape de solidification (E4), dans laquelle l'alliage métallique (3) est moulé pour obtenir une pièce moulée (2) conformée selon la surface texturée (s10), ladite pièce moulée (2) présentant au moins partiellement une structure amorphe, ladite pièce moulée (2) présentant une limite élastique strictement supérieure à 2200 MPa.

2. Procédé de fabrication selon la revendication 1, dans lequel lors de l'étape d'injection, l'alliage métallique (3) est un alliage comprenant une base métallique en Nickel, en Fer, en Cobalt, en Hafnium ou en Titane.

3. Procédé de fabrication selon la revendication 2, dans lequel l'alliage métallique (3) comprend un alliage Nickel/ Niobium.

**4.** Procédé de fabrication selon l'une quelconque des revendications 1 à 3, dans lequel lors de l'étape de mise à disposition (E1), la portion sommitale (13) de chaque structure miniature (11) présente un rayon de courbure (Rpm) inférieur à 300 $\mu$m.

**5.** Procédé de fabrication selon l'une quelconque des revendications 1 à 4, comprenant en outre une étape de découpe (E6), dans laquelle la pièce moulée (2) solidifiée issue de l'étape de solidification (E4) est découpée.

**6.** Procédé de fabrication selon l'une quelconque des revendications 1 à 5, comprenant en outre une étape de fusion (E2), mise en œuvre avant l'étape d'injection (E3), dans laquelle l'alliage métallique (3) est fondu par induction pour obtenir ledit alliage métallique (3) à l'état liquide.

**7.** Procédé de fabrication selon l'une quelconque des revendications 1 à 6, dans lequel lors de l'étape d'injection (E3), la cavité (10) du moule d'injection (1) est maintenue sous vide, ou en présence d'un gaz inerte comme l'argon.

**8.** Procédé de fabrication selon l'une quelconque des revendications 1 à 7, dans lequel lors de l'étape d'injection (E3), l'alliage métallique (3) à l'état liquide est injecté à une pression supérieure à 10 MPa et notamment supérieure à 50 MPa.

**9.** Procédé de fabrication selon l'une quelconque des revendications 1 à 8, dans lequel l'au moins un motif de structures miniatures (11) est un motif régulier de structures miniatures (11).

**10.** Procédé de fabrication selon la revendication 9, dans lequel lors de l'étape de mise à disposition (E1), le motif régulier de la surface texturée (s10) comprend l'alternance de creux et de pointes, lesdites pointes formant les portions sommitales (13).

**11.** Procédé de fabrication selon l'une quelconque des revendications 9 ou 10, dans lequel le motif régulier comprend une matrice de pyramides.

**12.** Procédé de fabrication selon l'une quelconque des revendications 1 à 11, dans lequel lors de l'étape de mise à disposition (E1), le motif de structures miniatures (11) comprend une face plane au niveau de laquelle la pluralité de structures miniatures (11) fait saillie régulièrement de ladite face plane.

**13.** Pièce moulée (2) en un verre métallique présentant au moins partiellement une structure amorphe, ladite pièce moulée (2) présentant à l'état solide, une limite élastique strictement supérieure à 2200 MPa, ladite pièce moulée (2) présentant en outre une surface de préhension texturée (s2) comprenant au moins un motif de structures de préhension miniatures (21), où chaque structure de préhension miniature (21) présente une portion creuse (23).

**14.** Pièce moulée (2) selon la revendication 13 présentant une contribution plastique à la flèche fp, supérieure à 0 mm, préférentiellement supérieure à 0,25 mm, plus préférentiellement supérieure à 0,60 mm, encore plus préférentiellement supérieure à 1 mm, ladite contribution plastique à la flèche fp étant déterminée lors d'un essai de flexion 3 points dans la direction de l'épaisseur pour un échantillon d'épaisseur 0,5 mm, de largeur 10 mm et de longueur 15 mm, une longueur entre appuis de 10 mm et une vitesse de traverse de 0,005 mm/s.

**15.** Plaquette de maintien (2a, 2b, 2c) pour outillage robotisé comprenant une pièce moulée (2) selon l'une quelconque des revendications 13 ou 14.

[Fig. 1]

(E1)

(E2)

3

(E3)

(E4)

1

11

23

2

10

s10   11   11   13

21   11   13   21

[Fig. 2]

1

11

11

13

s2

10

Rcm   Rpm

23

21

21

2

[Fig. 3]

[Fig. 4]

P0 Vs Rcm

▲ WC (elastic)          × WC (break)
● invention (elastic)    ○ invention (plastic)
■ Acier 440B Trempé (elastic)   □ Acier 440B Trempé (plastic)

[Fig. 5]

[Fig. 6]

[Fig. 7]

Europäisches
Patentamt

European
Patent Office

Office européen
des brevets

**RAPPORT DE RECHERCHE EUROPEENNE**

Numéro de la demande

EP 26 15 2296

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 9 724 450 B2 (OPIE DAVID [US]; NGUYEN TRANQUOC THEBAO [US] ET AL.) 8 août 2017 (2017-08-08) * colonne 3, ligne 34 - colonne 8, ligne 56; figures 1-4 * ----- | 1-15 | INV. B22D17/00 B22D17/22 A61B17/00 |
| X | CN 102 563 006 A (BYD CO LTD) 11 juillet 2012 (2012-07-11) * Machine Translation; alinéa [DetailedDescription]; figures 1-2 * ----- | 1-15 | |
| X | US 6 044 893 A (TANIGUCHI TAKESHI [JP] ET AL) 4 avril 2000 (2000-04-04) * alinéa [Detaileddescriptionoftheinvention]; figures 1-11 * ----- | 1-15 | |

| DOMAINES TECHNIQUES RECHERCHES (IPC) |
|---|
| B22D A61B |

Le présent rapport a été établi pour toutes les revendications

| Lieu de la recherche | Date d'achèvement de la recherche | Examinateur |
|---|---|---|
| La Haye | 18 mai 2026 | Desvignes, Rémi |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 26 15 2296

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-05-2026

| Document brevet cité au rapport de recherche | | Date de publication | Membre(s) de la famille de brevet(s) | | Date de publication |
|---|---|---|---|---|---|
| US 9724450 | B2 | 08-08-2017 | AU | 2003258298 A1 | 03-03-2004 |
| | | | EP | 1534175 A1 | 01-06-2005 |
| | | | EP | 2289568 A2 | 02-03-2011 |
| | | | US | 2006149391 A1 | 06-07-2006 |
| | | | US | 2012152412 A1 | 21-06-2012 |
| | | | US | 2012158151 A1 | 21-06-2012 |
| | | | WO | 2004016197 A1 | 26-02-2004 |
| CN 102563006 | A | 11-07-2012 | AUCUN | | |
| US 6044893 | A | 04-04-2000 | CN | 1202402 A | 23-12-1998 |
| | | | DE | 69806843 T2 | 13-03-2003 |
| | | | EP | 0875318 A1 | 04-11-1998 |
| | | | HK | 1016114 A1 | 29-10-1999 |
| | | | JP | 3808167 B2 | 09-08-2006 |
| | | | JP | H10296424 A | 10-11-1998 |
| | | | KR | 19980086714 A | 05-12-1998 |
| | | | TW | 503793 U | 21-09-2002 |
| | | | US | 6044893 A | 04-04-2000 |
| | | | US | 6189600 B1 | 20-02-2001 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **SURYANARAYANA, C ; INOUE, A**. Bulk Metallic Glasses. CRC Press, 2017

- **CHEUNG et al.** *Thermal and mechanical properties of Cu-Zr-Al bulk metallic glasses)*, 2007